# EUROPÄISCHE PATENTANMELDUNG

(11) **EP 0 829 237 A2**
(43) Veröffentlichungstag der Anmeldung: **18.03.1998**
(21) Anmeldenummer: 97810640.9
(22) Anmeldetag: 08.09.1997
(51) Int. Cl.: A61B 17/14, B23Q 5/00, B27B 19/00

(54) **Gerät zum Trennen von Materialien mit einem Trennwerkzeug auf einer oszillierend bewegten Antriebsachse**

(30) Priorität: 12.09.1996 CH 2239/96
(71) Anmelder: RICANA AG, 8600 Dübendorf (CH)
(72) Erfinder: Gunnewijk, Antonius G.M., 8714 Feldbach (CH)

(57) **Zusammenfassung**

Das Handgerät zum Trennen von Materialien weist ein Motormodul (2) mit Motorwelle (3) und ein separierbares Kopfmodul (12) mit Exzentervorrichtung (13) auf. Zwischen diesen separierbaren Modulen (2, 12) ist eine Kupplung (10) und ein elastisches Element (11) angeordnet. Eine Exzenterwelle (14) der Exzentervorrichtung ist der Kupplung gegenüberliegend einseitig gelagert. Über einen Exzenterzapfen (15) und ein Übertragungselement (17) wird eine oszillierende Antriebsachse (19) für ein Trennwerkzeug (6) angetrieben. Dies ergibt ein besonders leichtes, sicheres und einfach zu montierendes Trenngerät mit besseren Trenn- und Handhabungseigenschaften.

## Beschreibung

Die Erfindung bezieht sich auf ein Gerät zum Trennen von Materialien mit einem Trennwerkzeug auf einer oszillierend bewegten Antriebsachse gemäss Oberbegriff von Anspruch 1. Derartige Hand-Trenngeräte sind z.B. aus der CH 685 154 bekannt, speziell für medizinaltechnische Anwendungen, aber auch für andere anspruchsvolle, z.B. industrielle Anwendungen.

Die bisher bekannten Handtrenngeräte weisen noch verschiedene Nachteile auf, sie sind oft noch zu schwer und damit unhandlich und ermüdend im Einsatz, andererseits sind leichtgewichtige Geräte jedoch besonders stark Vibrationen ausgesetzt und verursachen Lärm. Je geringer das Gewicht, desto grösser das Problem mit Vibrationen und Lärm. Zudem sind für hohe Trennleistungen auch hohe bis sehr hohe Schwingungsfrequenzen erforderlich, was diese Probleme noch weiter verschärft. Hohe Trennleistungen erfordern zudem auch hohe spezifische Leistungen dieser Handgeräte, was wiederum deren Gewicht erhöht und was auch einen entsprechenden Energieaufwand erfordert, wodurch z.B. ein Batteriebetrieb von Handgeräten, welcher keine hinderliche Verbindungsleitung zum Netz benötigt, stark eingeschränkt wird. Ein weiterer wichtiger Aspekt liegt in der beeinträchtigten Sicherheit durch zu schwere und unhandliche Geräte.

Insgesamt ergibt all dies eine Verschlechterung der Handhabungs- und Trenneigenschaften. Das Problem liegt nun darin, gleichzeitig minimales Gewicht mit bestmöglicher Handlichkeit und möglichst hohe Laufruhe zu erreichen und auch optimale Trenneigenschaften zu erzielen. Als weitere Nachteile der bisherigen Handgeräte sollte die Herstellung, Montage wie auch der Reparaturaufwand vereinfacht und reduziert werden. Die Geräte sollten servicefreundlicher und kostengünstiger sein und die Einsatzbereitschaft sollte möglichst hoch liegen.

Es ist nun Aufgabe der Erfindung, diese Nachteile der bisherigen Geräte zu überwinden und im speziellen ein leichteres Gerät mit erhöhter Laufruhe, weniger Lärm auch bei sehr hohen Frequenzen, bei guter Handlichkeit, mit erweiterten Handhabungs- und Trenneigenschaften zu schaffen, welches zudem einfacher herzustellen und zu reparieren ist, welches einen geringeren Serviceaufwand und Betriebsaufwand erfordert und welches eine erhöhte Sicherheit aufweist und vor allem auch einen netzunabhängigen Betrieb ermöglicht.

Diese Aufgabe wird erfindungsgemäss gelöst mit einem Gerät nach Anspruch 1. Durch den modulmässigen Aufbau mit einer Kupplung und einem elastischen Element sowie mit der gegenüberliegenden einseitigen Lagerung der Exzentervorrichtung wird ein einfacher, leichter Aufbau möglich, mit welchem gleichzeitig auch Vibrationen und Lärm wesentlich reduziert werden. Diese kombinierte Wirkung ergibt die genannten wesentlichen Verbesserungen. Die erfindungsgemässen Trenngeräte eignen sich für anspruchsvolle Aufgaben und speziell auch im Bereich der Medizinaltechnik. Dies sind zum einen Anwendungen als Gipssägen, mit welchen auch schwierig zu bearbeitenden Faserverbundverbände (Light Cast) und auch Faserverbände mit elastischen Harzen (Soft Cast) gut zu trennen sind. Dank leichter Bauweise und besserer Handhabung ist es auch für feine Frauenhände möglich, diese bisher meist von Männern ausgeführten Arbeiten durchzuführen. Ganz wichtig ist die geringere Ermüdung dank leichterer Handhabung der erfindungsgemässen Trenngeräte, wodurch wiederum eine wesentlich höhere Sicherheit erreicht wird.

Eine weitere wichtige medizintechnische Anwendung liegt in der Chirurgie als Knochentrenngeräte, wo bestmögliche Handhabung und besonders schonende Trennschnitte, d.h. dünn, sauber und vor allem ohne Erwärmung und ohne Beeinträchtigung und Beschädigung des angrenzenden Knochenmaterials gefordert ist. Damit kann vor allem auch das grosse Problem der Thermonekrose überwunden werden.

Die erfindungsgemässen Trenngeräte ermöglichen aber auch im industriellen Bereich erweiterte Anwendungen. So sind z.B. neue Materialien und Verbundwerkstoffe bearbeitbar, welche sich mit bisherigen Trenngeräten noch kaum sauber trennen liessen. Dies betrifft beispielsweise Faserverbundwerkstoffe und Werkstoffe mit elastischen Elementen oder Leichtbauelemente mit Materialkombinationen wie Faserverbund mit Metallen, z.B. bei Faserverbundwerkstoffen Alu Wabenplatten. Dank der möglichen sehr hohen Frequenzen von z.B. 20'000 bis 40'000 Schwingungen pro Minute ist es sogar möglich, gummiartige Produkte oder elastische Materialien wie Leder und Textilien zu trennen.

Die abhängigen Patentansprüche betreffen vorteilhafte Weiterbildungen mit nochmals besseren Eigenschaften und Trennleistungen.

Im folgenden wird die Erfindung anhand von Figuren und Beispielen weiter erläutert. Es zeigen
- Fig. 1: ein erfindungsgemässes Trenngerät mit Motormodul und Kopfmodul,
- Fig. 2: eine Montage von Kopfmodul, Kupplung und Motormodul,
- Fig. 3: ein Trenngerät mit abgewinkelter Motorwelle,
- Fig. 4: ein Trenngerät mit abgewinkelter Antriebsachse,
- Fig. 5, 6: Beispiele elastischer Kupplungen,
- Fig. 7: einen geschlossenen Schwinghebel mit Antrieb eines Trennwerkzeugs,
- Fig. 8: einen gabelförmig offenen Schwinghebel,
- Fig. 9a: einen Schwinghebel aus Kohlefaser-Verbundwerkstoff,
- Fig. 9b: einen schickweisen Aufbau aus UD-CFK
- Fig. 10: einen Kniehebel als Übertragungselement,
- Fig. 11, 12: Schubstangen als Übertragungselemente.

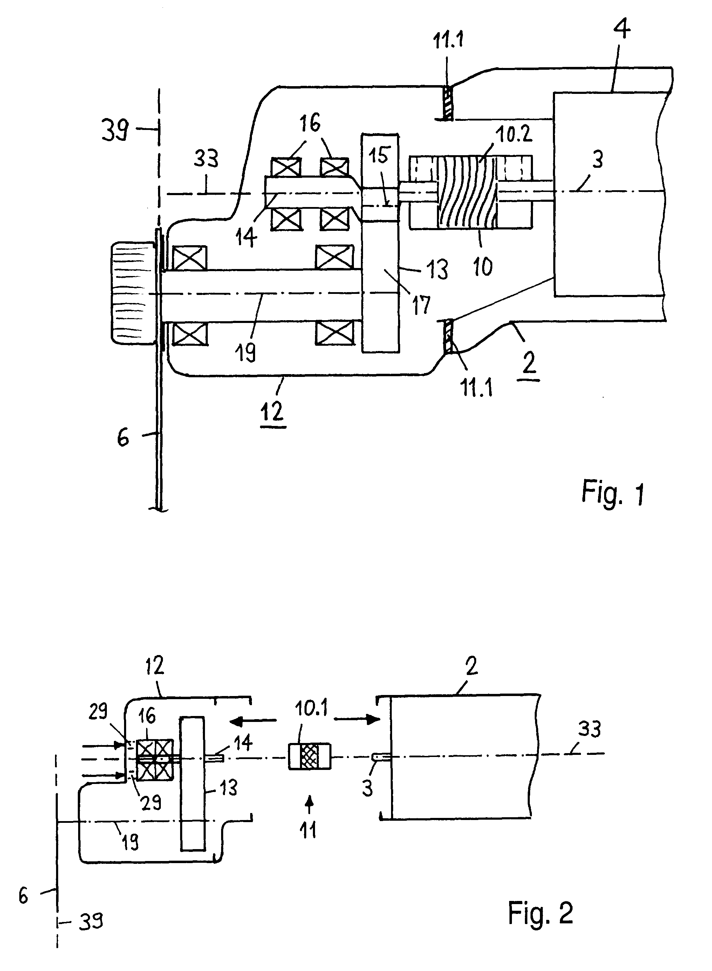

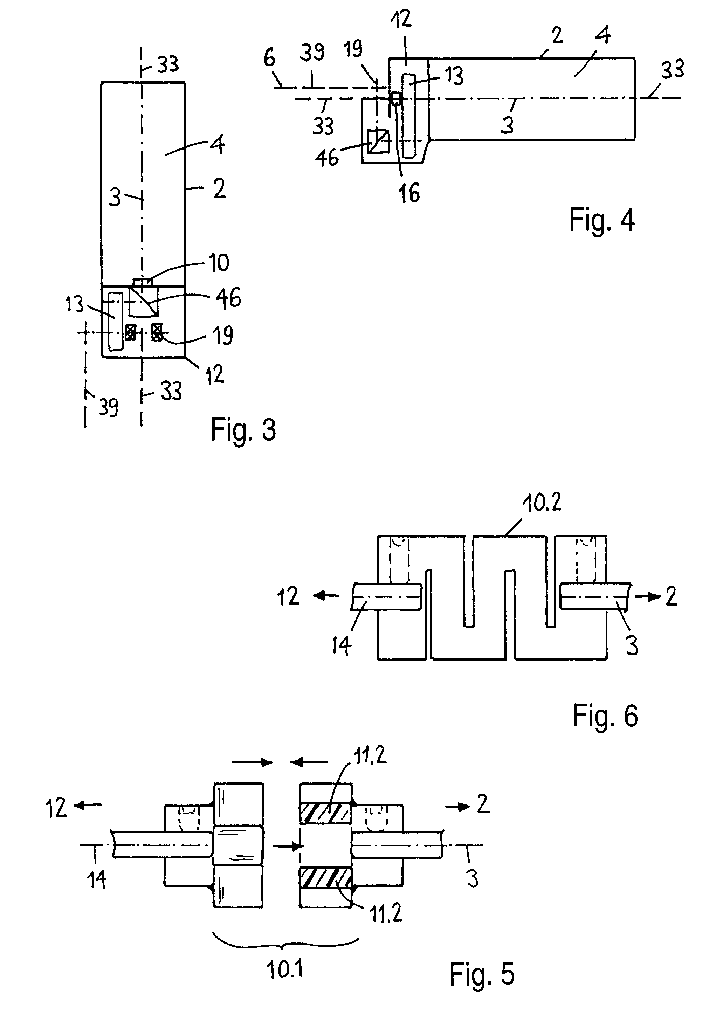

Fig. 1 zeigt ein erfindungsgemässes Handgerät zum Trennen von Materialien, bestehend aus einem Motormodul 2 mit Motor 4 und Motorwelle 3 und aus einem separierbaren Kopfmodul 12 mit einer Exzentervorrichtung 13, einer oszillierend bewegten Antriebsachse 19 und einem darauf aufgespannten Werkzeug 6, z.B. einem Sägeblatt. Die Module 2 und 12 sind an einer dazwischen liegenden Kupplung 10 separierbar ausgebildet, wobei zwischen Motormodul und Kopfmodul ein elastisches Element 11 montiert ist. In einer besonders einfachen und wirkungsvollen Ausführung ist die Kupplung 10 selber als elastisches Element ausgebildet, wie in den Fig. 5 und 6 beispielsweise weiter illustriert wird. Das elastische Element kann auch als Zwischenstück, z.B. als elastischer Ring 11.1, zwischen die beiden Gehäuse von Motormodul und Kopfmodul eingebaut werden. Mit diesem elastischen Element 11 werden oszillierende Schwingungen des Kopfmoduls vor allem bei anspruchsvollen Trennarbeiten vom Motormodul 2, welches als Handgriff dient, isoliert. Damit werden gleichzeitig auch die Trenneigenschaften verbessert. Im Kopfmodul 12 ist eine Exzenterwelle 14 mit einem Exzenterzapfen 15 einerseits über die Kupplung 10 mit der Motorwelle 3 verbunden und anderseits der Kupplung gegenüberliegend einseitig gelagert, z.B. hier mittels zweier Lager (z.B. Rillenkugellager) 16. Auf dem Exzenterzapfen 15 läuft ein Übertragungselement 17, welches die Antriebsachse 19 oszillierend bewegt.

Wie in Fig. 2 illustriert wird, ermöglicht diese erfindungsgemässe Bauart auch eine besonders einfache und damit kostengünstige Montage sowie wesentlich verbesserte Reparatur- und Serviceeigenschaften. Beide Module 2 und 12 sind zum einen je separat sehr einfach zu montieren mit sehr einfachem Aufbau, wobei im Kopfmodul 12 die ganze Exzentervorrichtung mit Lagerung und Übertragungselementen und die oszillierende Antriebsachse 19 einfach einlegbar sind, worauf zum andern auch die beiden Module auf einfachste Art an der Kupplung 10 unter Einschluss des elastischen Elements 11 zusammengesteckt und fixiert werden können. Der Ersatz allfälliger Verschleissteile, insbesondere von Lagern, aber auch einer elastischen Kupplung ist so ebenfalls auf sehr einfache Art rasch möglich. So kann z.B. ein Doppellager 16 zur Montage einfach ins Gehäuse hineingedrückt werden und zum Auswechseln durch zwei Ausstossöffnungen 29 mittels Stiften, welche direkt auf die Kugellageraussenringe wirken, wieder ausgestossen werden. Es ist auch möglich, auf bestimmte Anwendungen abgestimmte unterschiedliche Kupplungen und elastische Elemente einzusetzen (z.B. weichere oder härtere). In Fig. 1 ist beispielsweise eine Lagerung mit zwei separaten Kugellagern 16 und eine drehelastische Kupplung 10.2 dargestellt, während Fig. 2 ein Doppellager, z.B. ein Doppelkugellager, und eine elastische Klauenkupplung 10.1 darstellt. In beiden Fällen ist die Trennebene 39 für das oszillierende Werkzeug 6 rechtwinklig zur Motorwelle 3 und zur Geräteebene 33 angeordnet.

Die Fig. 3 und 4 zeigen Beispiele von Geräten, bei welchen die Trennebene 39 parallel zur Geräteebene 33 verläuft. Dies kann z.B. für tiefere Eingriffe, für grössere chirurgische Operationen mit grösseren Sägeblättern 6 vorteilhaft sein. In Fig. 3 wird die Motorwelle 3 durch ein Umlenkgetriebe oder Winkelgetriebe 46 z.B. in Form von Kegelrädern umgelenkt, so dass die Trennebene 39 im wesentlichen parallel zur Geräteebene 33 verläuft. Im Beispiel von Fig. 4 wird eine analoge Umlenkung der oszillierenden Antriebsachse 19 durch eine Umlenkung 46 realisiert, so dass die Trennebene 39 mit dem oszillierenden Werkzeug 6 ebenfalls parallel und nahe der Geräteebene 33 zu liegen kommt.

Um wie erwähnt, besonders gute Trenneigenschaften zu erreichen, sind meist hohe Drehzahlen bzw. Schwingungsfrequenzen erforderlich. Dazu werden hochdrehende Motoren mit hoher spezifischer Leistung, z.B. als AC- oder DC-Elektromotoren, eingesetzt, welche für kabelunabhängige Bedienung auch mit einem kleinen Batteriepack betreibbar sind, das z.B. am Gürtel der Bedienungsperson befestigt werden kann. Es sind aber auch andere Motoren mit hoher spezifischer Leistung einsetzbar, z.B. Druckluftmotoren besonders für sehr hohe Frequenzen bis über 1000 Hz.

Die Fig. 5 und 6 zeigen Beispiele von Kupplungen 10, welche als elastische Elemente 11 ausgebildet sind, um so zwischen der rotierenden Motorachse 3 und der oszillierend hin und herbewegten Antriebsachse 19 eine Lärm und Vibrationen reduzierende Schwingungsdämpfung zu realisieren. Das Beispiel in Fig. 5 zeigt eine beim Zusammenbau der Module 2 und 12 einfach zusammensteckbare Klauenkupplung 10.1 mit elastischen Einsätzen 1 1.2, welche auf den Achsen 3 und 14 befestigt wird.

Fig. 6 zeigt eine drehelastische Kupplung 10.2, z.B. mit einem metallischen Torsionsfederteil. Diese elastischen Kupplungen 10.1 und 10.2 sind sehr einfach auswechselbar wie auch das elastische Element 11.1 von Fig. 1. Durch entsprechende Dimensionierung und Wahl der elastischen Eigenschaften der Elemente 10 und 11 können auch optimale Trenn- und Handhabungseigenschaften, abgestimmt auf eine bestimmte Geräteart und -grösse wie auch auf Drehzahl und Einsatzbereich, erreicht werden.

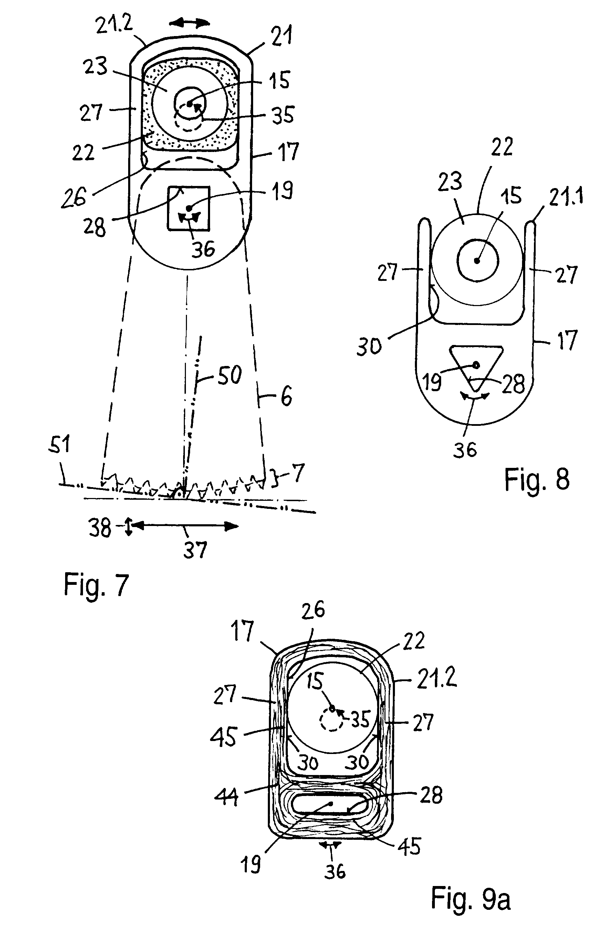

Beispiele von geeigneten leichten Exzentervorrichtungen 13 mit Übertragungselementen 17 werden in den Fig. 7 bis 12 illustriert. Dabei wird die rotierende Kreisbewegung 35 der Exzenterzapfen 15 durch das Übertragungselement 17 in eine oszillierende Hin- und Herbewegung 36 transformiert und auf die Antriebsachse 19 übertragen. Diese Übertragungselemente sind in den Fig. 7 bis 12 in Blickrichtung der Achsen 14 und 19 dargestellt.

Die Fig. 7 bis 9 zeigen Schwinghebel 21, in welchen ein auf dem Exzenterzapfen 15 gelagerter Gleitstein 22 bzw. ein Kugellager 23 abläuft und wobei die dadurch erzeugte Hin- und Herbewegung z.B. durch eine formschlüssige Ausnehmung 28 auf die Antriebsachse 19 übertragen wird. Diese Ausnehmungen 28 können z.B. quadratisch, dreiecksförmig, oval oder verrippt ausgebildet sein (Fig. 7 bis 12) oder der entsprechende Teil (21, 31, 41) der Übertragungselemente könnte auch fest mit der Achse 19 verbunden werden, z.B. durch Klebung.

Fig. 7 zeigt einen geschlossenen Schwinghebel 21.2 mit einer Ausnehmung 26, in welcher ein Gleitstein 22 mit Kugellager 23 auf und ab gleitet. Der Schwinghebel 21.1 im Beispiel von Fig. 8 ist gabelförmig offen ausgebildet, wobei hier das Kugellager 23 direkt als Gleitstein auf den Seitenstegen 27 abläuft. Diese Seitenstege 27 können relativ dünn ausgebildet sein, so dass sie eine vorgebbare, definierte Elastizität aufweisen. Damit ist ein weiteres Element zur Dämpfung von Lärm und Schwingungen wie auch zur Optimierung der Trenneigenschaften gegeben. Vorzugsweise sind die oszillierend bewegten Teile, im speziellen die Übertragungselemente 17, 21, 31, 42 der Fig. 7 bis 12, möglichst leicht ausgebildet. So kann mindestens ein solcher Teil aus Leichtbaumaterialien mit entsprechender Gleitpaarung an den Gleitflächen gefertigt sein.

Im Beispiel von Fig. 9a ist der Schwinghebel aus CFK Kohlefaser-Thermoplasten 44 hergestellt, wobei hier die Orientierung der C-Fasern im wesentlichen parallel zu den Ausnehmungen 26 und 28 und damit vor allem auch parallel zu den Gleitflächen 30 verläuft. Dies ergibt eine selbstschmierende Wirkung bei minimalem Abrieb und Verschleiss. Die Paarung der Gleitflächen, d.h. die Oberflächen am Gleitstein 22 bzw. am Kugellager 23 weisen dabei harte, glatte Metalloberflächen als Gegenstück zum CFK-Verbund auf, z.B. mit Hartverchromung.

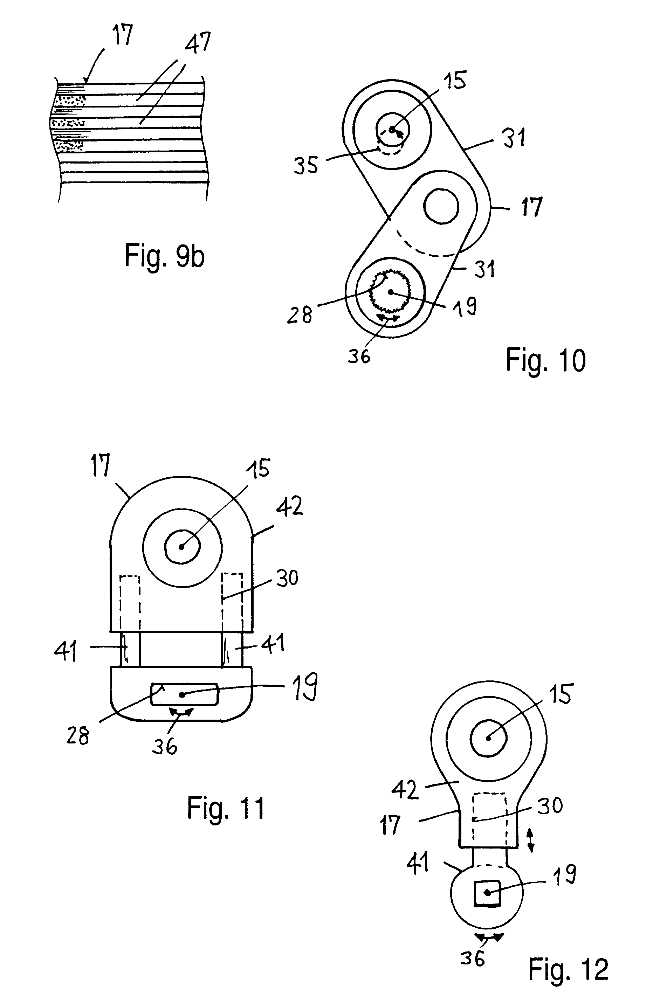

Fig. 9b zeigt als Beispiel einen schichtweisen CFK-Aufbau eines oszillierenden Übertragungselements 17 aus unidirektionalen (UD) Prepreg-Schichten 47. Diese UD Schichten sind abwechselnd mit unterschiedlichen Faserorientierungen (z.B. 0°, ± 45°, 90°) aufeinandergelegt, so dass wiederum vorzugsweise ein wesentlicher Anteil der Fasern (bzw. der UD-Schichten 47) parallel zu den Gleitflächen 30 verlaufen, dort z.B. mit einem Schichtaufbau von 90°, 90°, + 45°, 90°, 90°, - 45° Faserorientierung usw. Solche CFK-Thermoplastteile können z.B. durch orientiertes Einlegen der C-Fasern oder von Halbzeugschichten oder -elementen in ein Formwerkzeug und durch anschliessendes Heisspressen konsolidiert und gefertigt werden.

Beim Einsatz von Leichtmetallen als Leichtbaumaterial in oszillierenden Teilen wird vorzugsweise eine Gleitbeschichtung eingesetzt. Auch die Ausführungen der Übertragungselemente gemäss Fig. 10 bis 12 können mit Vorteil Leichtbauteile aufweisen.

In Fig. 7 ist weiter auch ein auf die oszillierende Antriebswelle 19 fest aufgespanntes Trennwerkzeug 6 dargestellt, hier in Form eines Sägeblatts mit ungeschränkter Verzahnung 7, d.h. die Seitenflächen der Verzahnung verlaufen parallel zur Blattebene. Solche z.B. aus der DE 32 22 339 oder der EP 637 433 bekannte Sägeblätter ergeben besonders gute Trenneigenschaften, vor allem auch für anspruchsvolle medizintechnische Aufgaben. Durch entsprechende exzentrische Einspannung (50) des Sägeblatts 6 bezüglich der oszillierenden Antriebsachse 19 oder durch nichttangentiale Anordnung (51) der Zahnreihe 7 am Sägeblatt kann eine besonders günstige Bewegungsform der Zähne erreicht werden, welche zusätzlich zur oszillierenden Bewegung 37 parallel zu den Zähnen auch eine Bewegungskomponente 38 in senkrechter Richtung dazu aufweist. Auch damit können die Trenneigenschaften erweitert und die Trennleistungen erhöht werden. Bei exzentrischer Einspannung geht die senkrecht zur Tangente der Zahnreihe 7 stehende Mittelachse 50 nicht durch den Drehpunkt der Antriebsachse 19, sondern verläuft z.B. in einem Abstand von Millimetern daran vorbei.

Fig. 10 zeigt eine Variante mit einem Kniehebel 31 als Übertragungselement und die Fig. 11 und 12 illustrieren Beispiele mit ein bzw. zwei Schubstangen 41, welche in einem Führungshülsenteil 42 ablaufen.

Auch diese oszillierenden Übertragungselemente können mit Vorteil aus CFK Thermoplast Verbundwerkstoffen gefertigt werden, um grosse Verbesserungen bez. Virbrationsdämpfung und Leistungserhöhung im Sinne der Aufgabe zu erzielen. Dies ist auch möglich mit einer anderen geeigneten Modulbauweise, z.B. auch mit beidseitiger Lagerung der Exzenterwelle 14. Geeigente Thermoplaste sind z.B. PEEK (Polyetheretherketone), PA (Polyamide), PC (Polycarbonate), PBT (Polybuthylenterephthalate) und PEI (Polyetherimide).

Auch diese Übertragungselemente nach Fig. 7 bis 12 sind gemäss Illustration der Montage nach Fig. 2 einfach zu montieren bzw. einzulegen und auszutauschen, was auch eine besonders hohe Servicefreundlichkeit ergibt.

Mit der Erfindung werden Trenngeräte geschaffen, welche hohe Frequenzen und besonders gute Trenneigenschaften ermöglichen, welche sehr leicht, energieeffizient und handlich sind und die auch dank reduzierten Vibrationen und Lärm besser, sicherer und ermüdungfrei zu bedienen sind. Sie erfordern einen geringeren Aufwand für Herstellung, Montage und Service und erschliessen einen erweiterten Einsatzbereich insbesondere auch für Anwendungen in der Medizinaltechnik.

Das Handgerät zum Trennen von Materialien weist ein Motormodul mit Motorwelle und ein separierbares Kopfmodul mit Exzentervorrichtung auf. Zwischen diesen separierbaren Modulen ist eine Kupplung und ein elastisches Element angeordnet. Eine Exzenterwelle der Exzentervorrichtung ist der Kupplung gegenüberliegend einseitig gelagert. Über einen Exzenterzapfen und ein Übertragungselement wird eine oszillierende Antriebsachse für ein Trennwerkzeug angetrieben. Dies ergibt ein besonders leichtes, sicheres und einfach zu montierendes Trenngerät mit besseren Trenn- und Handhabungseigenschaften.

## Patentansprüche

1. Handgerät zum Trennen von Materialien mit einem Trennwerkzeug auf einer oszillierend bewegten Antriebsachse (19), gekennzeichnet durch ein Motormodul (2) mit einer Motorwelle (3) und ein Kopfmodul (12) mit einer Exzentervorrichtung (13),
welche Module an einer dazwischenliegenden Kupplung (10) separierbar sind,
durch ein elastisches Element (11) zwischen Kopfmodul und Motormodul und durch eine Exzentervorrichtung mit einer mit der Kupplung verbundenen Exzenterwelle (14), welche der Kupplung gegenüberliegend einseitig gelagert ist (16),
mit einem Exzenterzapfen (15) und einem Übertragungselement (17), welches die oszillierende Antriebsachse (19) antreibt.

2. Gerät nach Anspruch 1, dadurch gekennzeichnet, dass die Kupplung (10) als elastisches Element ausgebildet ist.

3. Gerät nach Anspruch 2, gekennzeichnet durch eine elastische Klauenkupplung (10.1) oder eine dreh-elastische Kupplung (10.2).

4. Gerät nach einem der Ansprüche 1 bis 3, dadurch gekennzeichnet, dass das Übertragungselement als Schwinghebel (21) mit Gleitstein (22) ausgebildet ist.

5. Gerät nach Anspruch 4, dadurch gekennzeichnet, dass ein Kugellager (23) als Gleitstein dient.

6. Gerät nach Anspruch 4, gekennzeichnet durch einen offenen, gabelförmigen Schwinghebel (21.1).

7. Gerät nach Anspruch 4, gekennzeichnet durch einen geschlossenen Schwinghebel (21.2) mit einer Ausnehmung (26).

8. Gerät nach Anspruch 4, dadurch gekennzeichnet, dass der Schwinghebel dünne Seitenstege (27) mit definierter Elastizität aufweist.

9. Gerät nach einem der Ansprüche 1 bis 3, dadurch gekennzeichnet, dass das Übertragungselement als Kniehebel (31) ausgebildet ist.

10. Gerät nach einem der Ansprüche 1 bis 3, dadurch gekennzeichnet, dass das Übertragungselement eine oder zwei Schubstangen (41) und ein Führungshülsenteil (42) aufweist.

11. Gerät nach einem der Ansprüche 1 bis 10, gekennzeichnet durch ein Winkelgetriebe (46) an der Motorwelle (3) oder an der Antriebsachse (19), so dass die Trennebene (39) parallel zur Geräteebene (33) liegt.

12. Gerät nach einem der Ansprüche 1 bis 11, dadurch gekennzeichnet, dass mindestens ein oszillierend bewegter Teil aus Leichtbaumaterialien besteht.

13. Gerät nach Anspruch 12, dadurch gekennzeichnet, dass als Leichtbaumaterialien Leichtmetalle und eine Gleitbeschichtung eingesetzt sind.

14. Gerät nach Anspruch 12, dadurch gekennzeichnet, dass als Leichtbaumaterialien Kohlefaser-Thermoplast-Verbundwerkstoffe (44) mit Faserorientierung (45) parallel zu den Gleitflächen (30) eingesetzt sind.

15. Gerät nach Anspruch 14, dadurch gekennzeichnet, dass als Thermoplaste PEEK, PA, PC, PBT oder PEI eingesetzt sind.

16. Gerät nach Anspruch 14, dadurch gekennzeichnet, dass als Gleitpaarung zu den Kohlefaserthermoplasten harte, glatte Metalloberflächen vorgesehen sind.

17. Gerät nach einem der Ansprüche 1 bis 16, dadurch gekennzeichnet, dass leichte Sägeblätter mit ungeschränkter Verzahnung, d.h. mit parallelen Seitenflächen der Verzahnung, als Werkzeug (6) eingesetzt sind.

18. Gerät nach einem der Ansprüche 1 bis 17, dadurch gekennzeichnet, dass als Werkzeug Sägeblätter so angeordnet sind, dass zusätzlich zur oszillierenden Bewegung parallel zu den Zähnen (37) auch eine Bewegungskomponente in senkrechter Richtung (38) erzeugt wird.
